(19) Europäisches Patentamt
European Patent Office
Office européen des brevets

(11) EP 3 200 874 B1

(12) # EUROPÄISCHE PATENTSCHRIFT

(45) Veröffentlichungstag und Bekanntmachung des Hinweises auf die Patenterteilung:
**26.09.2018 Patentblatt 2018/39**

(21) Anmeldenummer: **15771140.9**

(22) Anmeldetag: **30.09.2015**

(51) Int Cl.:
***A61N 1/378*** *(2006.01)* ***A61N 1/362*** *(2006.01)*

(86) Internationale Anmeldenummer:
**PCT/EP2015/072556**

(87) Internationale Veröffentlichungsnummer:
**WO 2016/050845 (07.04.2016 Gazette 2016/14)**

(54) **VERFAHREN UND VORRICHTUNG ZUR VERSORGUNG EINES ELEKTRISCHE ENERGIE UMSETZENDEN IMPLANTATES MIT ELEKTRISCHER ENERGIE**

METHOD FOR SUPPLYING AN ELECTRICAL ENERGY-CONSUMING IMPLANT WITH ELECTRICAL ENERGY

PROCÉDÉ POUR ALIMENTER EN ÉNERGIE ÉLECTRIQUE UN IMPLANT CONVERTISSANT DE L'ÉNERGIE ÉLECTRIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.09.2014 DE 102014219815**

(43) Veröffentlichungstag der Anmeldung:
**09.08.2017 Patentblatt 2017/32**

(73) Patentinhaber: **Fraunhofer Gesellschaft zur Förderung der angewandten Forschung E.V.**
**80686 München (DE)**

(72) Erfinder:
- **SCHMID, Helmut**
  **72250 Freudenstadt (DE)**
- **ECKL, Wilhelm**
  **76227 Karlsruhe (DE)**

(74) Vertreter: **Gille Hrabal**
**Brucknerstrasse 20**
**40593 Düsseldorf (DE)**

(56) Entgegenhaltungen:
**WO-A1-2008/125866**
**WO-A2-2010/096774**
**US-A1- 2009 171 404**
**US-A1- 2009 281 600**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 200 874 B1

Printed by Jouve, 75001 PARIS (FR)

## Beschreibung

Die vorliegende Erfindung betrifft ein Verfahren zur Versorgung eines elektrische Energie umsetzenden Implantates mit elektrischer Energie mittels eines Freikolbengenerators.

Implantate, welche Energie zur Ausführung ihrer vorgesehenen Funktion benötigen, sind bekannt. Das am weitesten verbreitete Beispiel eines solchen Implantates ist der Herzschrittmacher. Bei einem solchen Herzschrittmacher wird der Impulsgenerator, der für das eigentliche Pacing (Schritt, Tempo machen) verantwortlich ist, mit einem Ein- oder Zwei-Elektroden-System verbunden, welches in die rechte Vorkammer (bei Sinusknoten-Impulsstörung) bzw. zusätzlich in die rechte Herzkammer (bei Störung der atrioventrikulären Überleitung) über die Vene eingeführt und verankert wird. Die Implantation erfolgt üblicherweise im oberen rechten Brustbereich (vom Patienten aus gesehen) unterhalb des Schlüsselbeins. Neuere Entwicklungen führten hierbei auch zur Miniaturisierung der bekannten Herzschrittmacher, so dass eine direkte Implantation in die Herzkammer möglich ist, wobei die Elektroden bereits Gerätebestandteile sind.

Allen diesen Geräten ist jedoch gemeinsam, dass sie elektrische Energie benötigen. Mittlerweile haben sich hierzu Lithium-Batterien etabliert, die eine Laufzeit von mehreren Jahren erreichen können. Danach ist jedoch eine Operation nötig, um den Herzschrittmacher, der verkapselt die Batterie enthält, zu ersetzen. Dabei müssen die Elektroden abgetrennt und eine neue Elektrodenverbindung hergestellt werden. Eine solche Operation stellt neben dem mit jeder Operation verbundenen Infektionsrisiko auch immer eine zusätzliche Belastung für den Patienten dar. Insbesondere die Narkose wie auch die psychische Belastung sind hierbei nicht zu unterschätzen.

Daher besteht ein grundsätzlicher Bedarf daran, eine nachhaltige Energieversorgung solcher Implantate bereitzustellen, um die oben genannten Risiken zu minimieren.

In diesem Zusammenhang wird beispielsweise in der WO 2008/125866 A1 eine Energiegewinnung innerhalb eines lebendigen biologischen Körpers vorgeschlagen. Dazu wird eine druckempfindliche Vorrichtung, welche ein Arbeitsfluid umfasst, mit einem elektrischen Generator verbunden und so eingestellt, dass Druckänderungen in dem Arbeitsfluid durch einen kolben in elektrische Energie umgewandelt werden. Dabei werden die Druckänderungen durch eine Vorrichtung, welche im rechten Ventrikel des Herzens oder aber auch in einem Blutgefäß angeordnet ist, genutzt. Dabei überträgt diese Vorrichtung die Blutdruckfluktuationen innerhalb des Blutkreislaufs in einem Säugetier auf das Arbeitsfluid. Das Arbeitsfluid wiederum treibt durch eine lineare Bewegung den elektrischen Generator an, wodurch elektrische Energie gewonnen wird, die einem Implantat zugeführt werden kann.

Nachteilig an diesem System ist, dass die Strömungspulsation des Blutes nur anteilig genutzt wird. Durch die zusätzliche Zwischenschaltung des Arbeitsfluids sinkt zudem der Wirkungsgrad. Außerdem bietet das Vorhandensein eines Arbeitsfluids auch immer ein zusätzliches Risiko bei Leckagen und ähnlichem.

In der US 3,563,245 wird ein System beschrieben, bei dem ein Anteil der Energie, welche durch die Muskelkontraktion des Herzens entsteht, einem Generator zugeführt wird und dieser wandelt im Anschluss diese Energie in elektrische Energie um. Auch hier wird eine Vorrichtung verwendet, welche drucksensitiv ist und durch Kontraktionen über ein Arbeitsfluid (hier ein Gas) eine Bewegungsenergie an den Generator überträgt. Damit ist die offenbarte Methode aber auch aus den oben genannten Gründen nachteilig.

Die US 2009/0171404 A1 offenbart unter anderem Systeme zur Erzeugung, Aufladung und Speicherung von elektrischer Energie, die für die Verwendung mit implantierten medizinischen Geräten geeignet sind und die mittels Generatoren durch die physikalische, chemische oder physiologische Aktivität wie die hämodynamischen Kräfte des Blutflusses oder durch das Schlagen des Herzens, beispielsweise über elektromagnetische Induktion, Leistung erzeugen können.

Des Weiteren existieren derzeit theoretische Überlegungen, mit einem piezoelektrischen Energieharvester den Herzdruck zu nutzen und somit elektrische Energie zu erzeugen. Bisher sind hierzu jedoch keine praktischen Umsetzungen bekannt. Jedoch ist auch bei diesen Überlegungen nachteilig, dass nur ein geringer Wirkungsgrad erzielt werden kann.

Die Gewinnung elektrischer Energie mittels eines Freikolbengenerators ist bereits seit vielen Jahren bekannt. Seine unterschiedlichen Ausgestaltungen sind daher dem Fachmann bekannt. Sie zeichnen sich insbesondere durch die kompakte Bauweise und das hohe Leistungs-Gewichts-Verhältnis aus. Ein Freikolbengenerator ist ein Lineargenerator, welcher eine geradlinige Bewegungsenergie in elektrische Energie umwandelt.

Ebenso kann der Freikolbengenerator invers auch als Freikolbenmotor eingesetzt werden. Beispielsweise ist aus der US 4,102,610 das Pumpen biologischer Flüssigkeiten mittels eines solchen Motors bekannt.

In der US 3,788,772 wird die Verwendung eines Freikolbenmotors zum Antrieb einer Herzmaschine während der Operation eines Patienten am Herzen vorgeschlagen. Hier übernimmt der Freikolbenmotor die Pumpfunktion des Herzens.

Eine ähnliche Pumpfunktion des Blutes durch einen Kolbenmotor, allerdings in miniaturisierter Form, wird auch in der US 5,879,375 sowie der WO 03/034893 A2 vorgeschlagen.

Jedoch ist die Problematik der nachhaltigen Energieversorgung von Implantaten bis heute noch nicht zufriedenstellend gelöst.

Ausgehend von diesem Stand der Technik liegt der vorliegenden Erfindung die Aufgabe zu Grunde, mindestens einen, bevorzugt alle Nachteile des Standes der Technik zu beheben. Insbesondere liegt der vorliegenden Erfindung die Aufgabe zu Grunde, ein Verfahren bereitzustellen, bei dem ein Implantat, welches elektrische Energie benötigt, nachhaltig mit elektrischer Energie versorgt wird. Dabei sollte der Wirkungsgrad des Systems mindestens genauso hoch, bevorzugt höher als der im Stand der Technik beschriebenen Systeme sein.

Diese Aufgaben wurden gelöst durch das erfindungsgemäße Verfahren, wie im Folgenden beschrieben.

Gemäß der vorliegenden Erfindung wird ein Verfahren zur Versorgung eines elektrische Energie umsetzenden Implantates mit elektrischer Energie bereitgestellt, welches die folgenden Schritte umfasst:

(a) Bereitstellen einer elektrischen Spannungsquelle mittels Erzeugung elektrischer Spannung durch die lineare Bewegung eines Kolbens **7** eines Freikolbengenerators **2,** wobei der Freikolbengenerator **2** einen Führungszylinder **6,** in dem sich ein Kolben **7** mit mindestens einem Permanentmagneten befindet, und mindestens eine Spule **8** umfasst, der Führungszylinder **6** in ein Blutgefäß **1** eingebracht ist und die mindestens eine Spule **8** sich außerhalb des Blutgefäßes **1** befindet,
der Kolben 7 innerhalb des Führungszylinders **6** des Freikolbengenerators **2** durch das periodisch pulsierende Blut im Blutgefäß **1** linear relativ in Richtung des jeweiligen Blutflusses **F** bewegt wird, und
in Abhängigkeit dieser linearen Bewegung des Kolbens **7** eine elektrische Spannung in der mindestens einen Spule **8** induziert wird, und
(b) Entnahme elektrischer Energie von der elektrischen Spannungsquelle aus Schritt (a) durch die Stromsteuerungseinheit des elektrische Energie umsetzenden Implantates.

**Verfahrensschritt (a)**

Der erfindungsgemäß eingesetzte Freikolbengenerator **2** umfasst einen Führungszylinder **6** und mindestens eine Spule **8.** Wie bereits oben ausgeführt, ist die allgemeine Konstruktionsweise eines Freikolbengenerators dem Fachmann bekannt.

Erfindungsgemäß können die Begriffe "umfassen" und "enthalten" in einer Ausführungsform auch "bestehen aus" bedeuten.

Der Führungszylinder **6** ist ein Hohlzylinder, welcher den Kolben **7** enthält. Dabei umfasst der Führungszylinder **6** bevorzugt ein Polymer, welches ausgewählt wird aus der Gruppe, bestehend aus polymeren fluorierten Kohlenwasserstoffen, Polysiloxanen, polymeren organofunktionalisierten Silanen und Copolymeren der vorgenannten Polymere. Hierbei kann der Führungszylinder **6** bevorzugt ein Polymer als Beschichtung umfassen, welches ausgewählt wird aus der Gruppe, bestehend aus polymeren fluorierten Kohlenwasserstoffen, Polysiloxanen, polymeren organofunktionalisierten Silanen und Copolymeren der vorgenannten Polymere. Bei den polymeren fluorierten Kohlenwasserstoffen handelt es sich bevorzugt um Fluorcarbone. Hier ist es weiterhin bevorzugt, dass die Fluorcarbone ausgewählt werden aus der Gruppe, bestehend aus Polyvinyltetrafluorid, Polyvinylidendifluorid und Polyvinylidenfluorid. Copolymere von Fluorcarbonen sind bevorzugt Perfluoralkoxy-Polymere (PFA) wie beispielsweise Copolymere aus Tetrafluor-Ethylen und Perfluor-Vinyl-Methyl-Ether.

Bei den Polysiloxanen handelt es sich bevorzugt um Polysiloxanharze, d. h. um Polysiloxane mit einem entsprechend hohen Verzweigungsgrad oder um lineare Polysiloxane, wie beispielsweise Hydrosilyl-Polydimethylsiloxan, welche anschließend vernetzt werden. Copolymere von Polysiloxanen umfassen bevorzugt Polyether-Polysiloxane, Polymethylsiloxan-Poylalkylsiloxan oder Polymethylsiloxan-Polyalkylsiloxan-Polyether.

Unter polymeren organofunktionalisierten Silanen versteht der Fachmann im Allgemeinen Polymere, welche die folgende allgemeine Struktur (I) umfassen:

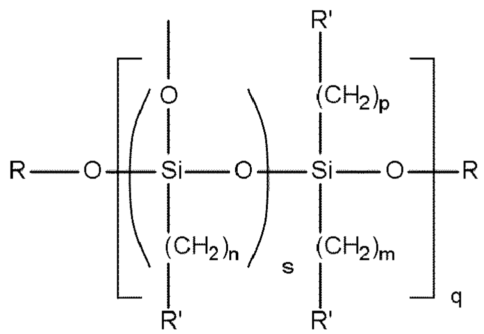

Damit stellen organofunktionalisierte Silane eine Untergruppe der Copolymere der Polysiloxane dar. Hierbei können die Gruppen R' insbesondere funktionale Gruppen sein, welche ausgewählt werden aus der Gruppe, bestehend aus $-C_6H_5$, -SH, $-NH_2$, $-(CF_2)_5CF_3$, $-N^+Me_3Cl^-$, $-O-CH_2-CH(O)CH_2$, $-CH=CH_2$, $-OC(O)CH=CH_2$ und $-OC(O)C(CH_3)=CH_2$. Die Zahlen n, m und p können bevorzugt unabhängig voneinander 1 bis 6 sein und s kann bevorzugt 0 bis 3 sein. R ist bevorzugt H oder-$CH_3$ und q ist bevorzugt 10000.

Das Polymer, welches der Führungszylinder **6** umfassen kann, kann des Weiteren weitere Bestandteile, wie beispielsweise einen niedermolekularen Vernetzer, enthalten. Die Notwendigkeit beziehungsweise Vorteilhaftigkeit der Einarbeitung solcher weiteren Bestandteile liegt im Wissen des Fachmanns.

Ebenso kann der Führungszylinder **6** zusätzlich noch eine (weitere) Beschichtung aufweisen. Diese Beschichtung befindet sich vorzugsweise an der Außenseite des Führungszylinders **6,** d. h. der Seite, welche mit Blut in Kontakt kommt. Beschichtungen oder auch Oberflächenbehandlungen, um diese Außenseite des Führungszylinders an die Einsatzbedingungen, insbesondere den Kontakt mit Blut anzupassen, sind dem Fachmann bekannt.

Bevorzugt weist der Führungszylinder eine Wandstärke von 0,2 bis 1,8 mm, besonders bevorzugt von 0,5 bis 1,5 mm und ganz besonders bevorzugt von 0,9 bis 1,1 mm auf. Bevorzugt weist der Führungszylinder **6** einen Außendurchmesser von 2,5 bis 7,5 mm, bevorzugt 4 bis 6 mm, besonders bevorzugt von im Wesentlichen 5 mm auf. Bevorzugt weist der Führungszylinder eine Länge (relativ zum Blutfluss **F**) von 15 bis 55 mm, besonders bevorzugt 25 bis 45 mm, des Weiteren bevorzugt 30 bis 40 mm, ganz besonders bevorzugt im Wesentlichen 35 mm auf.

Der Führungszylinder **6** weist bevorzugt aufsetzbare Kappen auf, welche zum Ende hin, d. h. an der Deckfläche und der Grundfläche des Hohlzylinders, verjüngt sind (siehe Figur 1). Dadurch wird die Einführung des Führungszylinders in das Blutgefäß **1** mittels Katheter vereinfacht. Ebenso wird der Kolben 7 dadurch im Führungszylinder **6** gehalten und dadurch in seinem Bewegungsraum begrenzt. Die aufsetzbaren Kappen sind dabei bevorzugt abnehmbar montiert (z. B. durch Steck-, Clip- oder Schnappverbindungen). Bevorzugt sind die inneren Enden des Führungszylinders **6** formschlüssig an den Kolben **7** angepasst. Beispielsweise weisen die inneren Enden, d. h. die innere Deckfläche und Grundfläche des Hohlzylinders, bevorzugt eine konkave Form auf.

Der Führungszylinder **7** wird bevorzugt im Wesentlichen axialsymmetrisch im Blutgefäß (1) angeordnet. Im Sinne der vorliegenden Erfindung wird der Ausdruck "im Wesentlichen" verwendet, um geringe Abweichungen grundsätzlich zuzulassen. Bevorzugt betragen diese Abweichungen jedoch nicht mehr als 25 %, besonders bevorzugt nicht mehr als 10 % und ganz besonders bevorzugt nicht mehr als 5 % des eigentlich angegebenen Wertes. Insgesamt befindet sich somit der Führungszylinder **7** bevorzugt in der Mitte des Blutgefäßes **1,** so dass das Blut im Blutgefäß **1** in einem Zylinder um die Außenwand des Führungszylinders **7** bis hin zur Innenwand des Blutgefäßes **1** in Richtung **F** strömen kann (vgl. Figur 1).

Dabei ist es des Weiteren bevorzugt, dass der Führungszylinder **7** von einem rohrförmigen Gittergerüst **4,** welches mindestens eine Halterung **5** zum Halten des Führungszylinders aufweist, gehalten wird. Bei dem rohrförmigen Gittergerüst **4** handelt es sich bevorzugt um einen Stent. Dieser ist aus dem Stand der Technik bekannt. Ein solches rohrförmiges Gittergerüst **4** dient bevrozugt der Einführung des Führungszylinders in ein Blutgefäß mittels dem Fachmann bekannter Kathetertechnik (z. B. Kathetertechnik für Ballon-Dilatation, wie für die Perkutane Transluminale Coronare Angioplastie entwickelt). Zudem kann dieses rohrförmige Gittergerüst **4** des Weiteren auf bekannte Weise mit einem Ballon expandiert werden und dadurch den Führungszylinder **7** an der gewünschten Stelle in einem Blutgefäß **1** zu fixieren. Dies hat insbesondere den Vorteil, dass der Einsatz des erfindungsgemäßen Freikolbengenerators **2** insgesamt minimalinvasiv durchgeführt werden kann. Dies führt dazu, dass die Belastung des Patienten durch den Einsatz des erfindungsgemäßen Freikolbengenerators **2** geringer ist als bei den bisher bekannten Techniken. Das Infektionsrisiko wird dadurch reduziert.

Erfindungsgemäß ist der Führungszylinder (ggf. gemeinsam mit dem rohrförmigen Gittergerüst) bereits in ein Blutgefäß eingebracht. Damit wird erfindungsgemäß der Schritt der Einführung des Führungszylinders in das Blutgefäß ausgeschlossen.

Bevorzugt umfasst das rohrförmige Gittergerüst **4** eine Nickel-Titan-Legierung. Besonders bevorzugt umfasst das rohrförmige Gittergerüst **4** Nitinol. Ganz besonders bevorzugt besteht das rohrförmige Gittergerüst **4** aus Nitinol.

Des Weiteren weist das rohrförmige Gittergerüst **4** mindestens eine Halterung **5** zum Halten des Führungszylinders **7** auf. Solche Halterungen **5** sind bevorzugt so ausgebildet, dass der Blutfluss **F** zwischen der Außenwand des Führungszylinders **7** und der Innenwand des Blutgefäßes möglichst wenig behindert wird. Dabei weist das rohrförmige Gittergerüst **4** so viele Halterungen auf, wie benötigt werden, um den Führungszylinder **7** fest zu halten, d. h. gegen eine Verschiebung mit dem Blutstrom **F** zu schützen, und so wenige Halterung wie möglich, um den Einfluss auf den Blutstrom **F** gering zu halten. Besonders bevorzugt ist diese mindestens eine Halterung **5** eine Verstrebung zwischen dem rohrförmigen Gittergerüst **4** und dem Führungszylinder **7.** Ganz besonders bevorzugt umfasst oder besteht die mindestens eine Halterung **5** aus dem gleichen Material wie das rohrförmige Gittergerüst **4.**

Der Führungszylinder **6** umfasst den Kolben 7. Dabei ist es bevorzugt, dass der Kolben **7** formschlüssig im Führungszylinder **6** geführt wird. Die Formschlüssigkeit bezieht sich hierbei zunächst auf die Form des Kolbens **7** längs relativ zum Blutfluss **F.** Damit weist der Kolben bevorzugt eine zylindrische Form auf.

Bevorzugt weist der Kolben **7** im Wesentlichen eine gerade zylindrische Form mit einer Kreisgrundfläche auf und an der Grundfläche und Deckfläche des Zylinders ist jeweils eine im Wesentlichen halbkugelförmige Kappe formschlüssig aufgesetzt (vgl. Figur 1). Dabei ist es besonders bevorzugt, dass mindestens eine der im Wesentlichen halbkugelförmigen Kappen mindestens eine Öffnung aufweist. Bevorzugt weisen beide im Wesentlichen halbkugelförmigen Kappen mindestens eine Öffnung auf.

Es hat sich überraschenderweise herausgestellt, dass diese auf den Kolben **7** aufgesetzten Kappen dazu geeignet sind, kontrolliert Wirkstoffe freizusetzen. Damit kann die mindestens eine Öffnung der im Wesentlichen halbkugelförmigen Kappe zur kontrollierten Abgabe mindestens eines Wirkstoffes verwendet werden. Bevorzugt wird dieser Wirkstoff ausgewählt aus der Gruppe, bestehend aus Wirkstoffen, welche gängigerweise intravenös verabreicht werden. Insbesondere wird der Wirkstoff ausgewählt aus der Gruppe, bestehend aus Analgetika, Antibiotika und Antiinfektiva. Bevorzugt wird dabei mindestens ein Wirkstoff abgegeben, welcher die Thrombenbildung minimiert. Besonders bevorzugt wird mindestens ein Wirkstoff abgegeben, welcher ausgewählt wird aus der Gruppe, bestehend aus Heparin, Acetyl-Salicylsäure, Doxorubicin, TNT und beliebigen Kombinationen davon. Hierbei ist die Verwendung von TNT vorteilhaft, weil dieses blutdruckregulierend wirkt. Ebenso ist die Verwendung von Heparin und/oder Acetyl-Salicylsäure vorteilhaft, weil diese blutverdünnend wirken.

Die Kinetik der Wirkstoffabgabe kann dabei durch eine Nanoperforation, bevorzugt in der Zylinderachse des Kolbens **7,** gesteuert werden. Die Nanoperforation weist dabei bevorzugt eine definierte kreisförmige Querschnittsfläche auf. Dabei ist der Durchmesser dieser Fläche bevorzugt 5 bis 25 nm, besonders bevorzug 10 bis 20 nm und ganz besonders bevorzugt 14 bis 16 nm. Bevorzugt kann diese Nanoperforation durch bekannte lithographische Verfahren in die Kappen eingebracht werden. Somit kann erfindungsgemäß zusätzlich auch ein Nano-Carrier, Nano-Velo oder ein Nano-Container bereitgestellt werden.

Dabei ist es besonders bevorzugt, dass mindestens ein Enden des Führungszylinders 6 abnehmbar ist (beispielsweise durch eine Steck- oder Schraubverbindung). Dadurch ist es möglich, dass ein Austausch des Kolbens **7** nach der vollständigen Wirkstoffabgabe gegen einen neuen Kolben **7** minimalinvasiv durchgeführt werden kann. Auf diese Weise ist ebenfalls eine einfache Wirkstoffversorgung möglich.

Bevorzugt ist der Kolben **7** mit einer Polymerhülle ummantelt. Besonders bevorzugt enthält die Ummantelung des Kolbens **7** ein Polymer, welches ausgewählt wird aus der Gruppe, bestehend aus polymeren fluorierten Kohlenwasserstoffen, Polysiloxanen, polymeren organofunktionalisierten Silanen und Copolymeren der vorgenannten Polymere. Diese Polymere wurden oben bereits näher beschrieben und insbesondere gelten die oben genannten Bevorzugungen auch für die bevorzugten Materialien der Polymerhülle des Kolbens **7.**

Das Polymer, welches die Hülle des Kolbens **7** umfassen kann, kann des Weiteren weitere Bestandteile, wie beispielsweise einen niedermolekularen Vernetzer, enthalten. Die Notwendigkeit beziehungsweise Vorteilhaftigkeit der Einarbeitung solcher weiteren Bestandteile liegt im Wissen des Fachmanns.

Bevorzugt weist diese Ummantelung eine Wandstärke von 0,01 bis 0,2 mm auf, besonders bevorzugt 0,05 bis 0,15 mm, ganz besonders bevorzugt von 0,09 bis 0,1 mm. Dadurch wird gewährleistet, dass eine hohe Biokompatibilität, chemische Stabilität und gute Gleiteigenschaften simultan erzielt werden.

Der Kolben **7** weist mindestens einen Permanentmagneten auf. Dies bedeutet, dass er einen Permanentmagneten umfassen kann oder aus diesem bestehen kann. Besonders bevorzugt ist der Kolben **7** ein zylindrischer Permanentmagnet mit zwei, wie oben beschriebenen, im Wesentlichen halbkugelförmig aufgesetzten Kappen. Damit ist dieses Gesamtsystem bevorzugt mit der oben beschriebenen Ummantelung versehen.

Besonders bevorzugt ist der Permanentmagnet ein Seltenerd-Supermagent. Ganz besonders bevorzugt ist der Permanentmagnet die ferromagnetische intermetallische Verbindung $Nd_2Fe_{14}B$. Es hat sich herausgestellt, dass diese die Anforderungen nach einer hohen magnetischen Energiedichte $W_m = ½ B*H$ [$VAs/m^3 = J/m^3$] durch gleichzeitig hohe Werte der Remanenz-Flussdichte $B_r$ von 1,4 T und der Koerzitiv-Feldstärke $H_c$ von 2000 kOe erfüllt und damit besonders geeignet als Material für den erfindungsgemäßen Kolben **7** ist.

Der Führungszylinder **6,** welcher den Kolben **7** umfasst, ist gegebenenfalls mit dem rohrförmigen Gittergerüst **4** als Gesamtsystem **3** in ein Blutgefäß **1** eingebracht. Dieses Blutgefäß **1** ist bevorzugt eine Vene.

Dabei ist es besonders bevorzugt, dass das Gesamtsystem **3** und die mindestens eine Spule **8** im herznahen rechten Venenbereich eingebracht werden. Als "herznah" wird hier insbesondere ein Bereich verstanden, bei dem der Venendurchmesser (Innendurchmesser) zwischen 5 bis 15 mm, bevorzugt 8 bis 12 mm, besonders bevorzugt im Wesentlichen 10 mm beträgt. Die Wandstärke der Vene beträgt hier insbesondere im Wesentlichen 1,5 mm.

Diese Position des Freikolbengenerators **2** ist deshalb bevorzugt, da hier eine hohe Strömungsgeschwindigkeit und auch eine ausreichende Rückströmungsgeschwindigkeit vorliegen. Bevorzugt liegt am Transplantationsort eine Strömungsgeschwindigkeit von 0,5 bis 1,5, m/s, besonders bevorzugt von 0,8 bis 1,2 m/s und ganz besonders bevorzugt im Wesentlichen 1 m/s vor. Dabei ist die Periodendauer bevorzugt im Wesentlichen 1 s. Des Weiteren ist dabei bevorzugt, dass die pulsierende Rückströmung eine Strömungsgeschwindigkeit von 0,1 bis 0,5 m/s, besonders bevorzugt von 0,2 bis 0,4 m/s und ganz besonders bevorzugt von im Wesentlichen 0,3 aufweist.

Es ist auch möglich, den erfindungsgemäßen Freikolbengenerator **2** an einem anderen Ort einzusetzen. Unterstützend kann noch eine mechanische Feder **9** in den Führungszylinder **6** eingebracht sein. Dies ist insbesondere vorteilhaft, wenn dieser andere Implantationsort eine sehr geringe oder sogar zu geringe pulsierende Rückströmung aufweist. Diese mechanische Feder **9** bewirkt somit eine unterstützende Rückbewegung des Kolbens **7** innerhalb des Führungszylinders **6.** Bevorzugt ist diese Feder mit Polymer beschichtet. Dabei handelt es sich bevorzugt um ein Polymer, welches ausgewählt wird aus der Gruppe, bestehend aus polymeren fluorierten Kohlenwasserstoffen, Polysiloxanen, polymeren organofunktionalisierten Silanen und Copolymeren der vorgenannten Polymere. Diese Polymere wurden oben bereits näher beschrieben und insbesondere gelten die oben genannten Bevorzugungen auch für die hier bevorzugten Materialien.

Der Freikolbengenerator **2** umfasst des Weiteren mindestens eine Spule **8.** Diese Spule **8** wird außerhalb des Blutgefäßes **1** angebracht, jedoch im Wesentlichen an der Stelle, an der das Gesamtsystem **3** sich innerhalb des Blutgefäßes **1** befindet. Dabei ist es bevorzugt, dass das Gesamtsystem **3** innerhalb des Blutgefäßes **1** und die mindestens eine Spule **8** sich achsensymmetrisch in Bezug auf das Blutgefäß **1** erstrecken (siehe Figur 1).

Die Spule **8** befindet sich außerhalb des Blutgefäßes **1.** Dies bedeutet, sie befindet sich nicht im Blutfluss **F,** sondern auf der anderen Seite der Gefäßwandung, wo kein Blutfluss stattfindet. Bevorzugt ist die Spule um das Blutgefäß **1** herum platziert.

Bevorzugt ist die mindestens eine Spule **8** in einen Mantel oder Spulenträger gebettet. Dabei besteht der Mantel bevorzugt aus mindestens zwei (Halb)schalen, welche so miteinander verbunden werden können, dass sie dicht schließen und gleichzeitig die Spulenwindungen miteinander kontaktieren. Damit ist es möglich, minimalinvasiv die mindestens eine Spule **8** um ein Blutgefäß **1** herum zu platzieren. Bevorzugt beträgt der Innendurchmesser der mindestens einen Spule **8** 8 bis 18 mm, besonders bevorzugt 11 bis 15 mm, ganz besonders bevorzugt im Wesentlichen 13 mm. Somit wird sichergestellt, dass das Blutgefäß **1** nicht beschädigt wird. Die Länge dieses Mantels (in Richtung relativ zum Blutfluss F) beträgt bevorzugt 20 bis 60 mm, besonders bevorzugt 30 bis 50 mm, des Weiteren bevorzugt 35 bis 45 mm, ganz besonders bevorzugt im Wesentlichen 40 mm.

Bevorzugt umfasst der Mantel ein Polymer, welches ausgewählt wird aus der Gruppe, bestehend aus polymeren fluorierten Kohlenwasserstoffen, Polysiloxanen, polymeren organofunktionalisierten Silanen und Copolymeren der vorgenannten Polymere. Diese Polymere wurden oben bereits näher beschrieben und insbesondere gelten die oben genannten Bevorzugungen auch für die bevorzugten Materialien des Mantels.

Das Polymer, welches der Mantel umfassen kann, kann des Weiteren weitere Bestandteile, wie beispielsweise einen niedermolekularen Vernetzer, enthalten. Die Notwendigkeit beziehungsweise Vorteilhaftigkeit der Einarbeitung solcher weiteren Bestandteile liegt im Wissen des Fachmanns.

Bevorzugt ist um den erfindungsgemäßen Freikolbengenerator **2** ein Gehäuse zur magnetischen Abschirmung vorgesehen.

Diese oben räumlich-körperlich beschriebenen Bauteile bilden im Wesentlichen den erfindungsgemäßen Freikolbengenerator **2.** Im Folgenden wird nun seine Funktionsweise an Hand der Figur 1 beschrieben.

Die zeitlichen Verläufe von Strömungsgeschwindigkeit und Druck der periodisch pulsierenden (instationären) Blutströmung weisen insbesondere an der oben angegebenen bevorzugten Position Größenordnungen auf, welche zum Antrieb des erfindungsgemäßen Freikolbengenerators **2** ausreichen.

Fließt das Blut in Richtung des Blutflusses **F** zunächst in eine Richtung, so wird der Kolben **7** im Führungszylinder **6** in die gleiche Richtung wie der Blutfluss verschoben. Kommt es durch die Pulsation zu einem Rückfluss in die entgegengesetzte Richtung, so wird der Kolben **7** im Führungszylinder **6** ebenfalls wieder in die entgegengesetzte Richtung bewegt. Dies kann, wie oben beschrieben, durch eine zusätzliche mechanische Feder unterstützt werden (siehe Figur 2). Insgesamt wird also die Pulsation des Blutes genutzt, um eine lineare Hin- und Zurückbewegung (relativ zum Blutfluss **F**) des Kolbens **7** innerhalb des Führungszylinders **6** zu bewirken. Der Blutfluss wird hierbei entweder vom Herzen selbst und/oder aber von einem den Blutfluss unterstützenden Element, beispielsweise einem Herzschrittmacher, bewirkt, wobei das den Blutfluss unterstützende Element zunächst mit einer aufgeladenen Batterie verbunden ist.

Für die beschriebene Bewegung wird daher die Pulsation des Blutes und nicht, wie im Stand der Technik beschrieben, die Druckdifferenz ausgenutzt. Dies führt erfindungsgemäß dazu, dass der Wirkungsgrad des Freikolbengenerators höher ist als der Generatoren des Standes der Technik. Durch direkte Übertragung der Strömungskräfte des Blutes kann daher eine effektive Bewegungsenergie gewonnen werden. Dabei ist zudem kein zusätzliches Arbeitsfluid notwendig, wodurch weitere Risiken bei einer Operation und vor allem Risiken einer Leckage vermieden werden.

Das grundsätzliche Prinzip der Umwandlung der Bewegungsenergie in elektrische Energie ist dem Fachmann bekannt. In Abhängigkeit der linearen Bewegung des magnetischen Kolbens **7** wird in der mindestens einen Spule **8** eine elektrische Spannung induziert. Insgesamt wird somit eine elektrische Spannung bereitgestellt, die dem erfindungsgemäßen Freikolbengenerator **2** entnommen werden kann.

Grundsätzlich behindert der erfindungsgemäße Freikolbengenerator **2** die freie Blutströmung **F.** es konnte jedoch überraschenderweise gezeigt werden, dass mit dem erfindungsgemäßen Verfahren für den Patienten keine gesundheitliche Beeinträchtigungen zu erwarten sind.

Dabei ist es bevorzugt, dass ein anteiliger Restdurchmesser des Blutgefäßes **1** die folgende Anforderung erfüllt:

$$1 > x_{rd} = d/D \geq \text{(größer gleich) } 0{,}5,$$

worin

$x_{rd}$ für den anteiligen Restdurchmesser des Blutgefäßes **1,** welcher der Strömung des Blutes an der Stelle des Blutgefäßes **1,** an der der Führungszylinder **6** des Freikolbengenerators **2** fixiert ist, zur Verfügung steht, steht,

d für den noch verbleibenden Durchmesser des Blutgefäßes **1,** welcher der Strömung des Blutes an der Stelle des Blutgefäßes **1,** an der der Führungszylinder **6** des Freikolbengenerators **2** fixiert ist, zur Verfügung steht, steht und

D für den Gesamtdurchmesser des Blutgefäßes **1** steht, wobei alle Durchmesser Innendurchmesser des Blutgefäßes **1** sind.

Diese Anforderung konnte durch Berechnungen belegt werden. Dabei wurde von einer venösen Blutströmung ausgegangen. Vereinfachend wurde Blut als einphasige Flüssigkeit mit einer Dichte von 1060 $kg/m^3$ angesehen und als eine newtonsche Flüssigkeit konstanter Viskosität betrachtet. Dabei wurde vorausgesetzt, dass sich die bekannten zahlreichen und komplexen Abweichungen von der Realität in der Summe weitgehend ausmitteln. Die Strömung in einem starren Rohr wurde zu Grunde gelegt.

Das Strömungshindernis wurde als von der Wandung her eindringendes axialsymmetrisches Objekt mit der Form einer Halbwelle angenommen, wobei ein Längenverhältnis und Einschnürungsverhältnis bezogen auf den freien Durchmesser D festgelegt wurde. Mit einem Formfaktor von l/D = 3,5 (wobei l die Länge des Hindernisses darstellt) ließen sich eine Reynoldszahl von ca. 530 und eine Frequenzzahl der instationären Strömung $S = (\rho * f * D^2)/\eta$ von ca. 10 abschätzen.

Die zentrale Durchmesserreduktion durch den Führungszylinder **6** ist dabei im Vergleich zur peripheren Durchmesserreduktion durch das rohrförmige Gittergerüst **4** in der Auswirkung auf die Reduktion der Querschnittsfläche deutlich geringer.

Das Ergebnis der mathematischen Modellierung basiert auf den Bewegungsgleichungen und der Kontinuitätsgleichung. Durch den Generator wird die Strömungsgeschwindigkeit des Blutes insgesamt erhöht und die maximale Strömungsgeschwindigkeit im Verhältnis zur mittleren Strömungsgeschwindigkeit um den Faktor 1,5 vergrößert. Dies bedeutet, dass die Unterschiede der Strömungsgeschwindigkeiten ebenfalls zunehmen.

Unter Beachtung des medizinischen Basiswissens kann gefolgert werden, dass in einem Bereich von $1 > x_{rd} = d/D \geq 0{,}5$ eine gesundheitliche Beeinträchtigung unwahrscheinlich ist.

**Verfahrensschritt (b)**

Erfindungsgemäße erfolgt in Schritt (b) die Entnahme elektrischer Energie von der elektrischen Spannungsquelle aus Schritt (a) durch die Stromsteuerungseinheit des elektrische Energie umsetzenden Implantates.

Dazu sind an der Spule 8 Endkontakte angebracht. Sie dienen als Schnittstelle zu einer Verkabelung, die zum elektrische Energie umsetzenden Implantat führt. Im Implantat selbst wird die elektrische Energie bevorzugt nach elektronischer Stabilisierung bevorzugt zur Aufladung bzw. Frischhaltung des Akkumulators verwendet, mit dem das Implantat betrieben wird.

Die Stromabführung und Kontaktierung des elektrische Energie umsetzenden Implantats geschieht nach dem Stand der Technik, beispielsweise über verfügbare Leitungs- und Verbindungselemente. Dabei existieren Stromregulierungselemente oder Stromsteuerungseinheiten, die das Implantat entsprechend den Anforderungen mit elektrischer Energie versorgen.

Bevorzugt ist das elektrische Energie umsetzende Implantat ein Herzschrittmacher.

Insgesamt kann somit durch das erfindungsgemäße Verfahren eine nachhaltige Versorgung eines Implantates mit elektrischer Energie gewährleistet werden. Dabei können insbesondere Operationen zum Austausch entladener Batterien vermieden werden. Erfindungsgemäß kann jedoch ein Austausch des Kolbens **7** zur Auffüllung der Wirkstoffdepots vorgesehen sein. Dieser Eingriff kann jedoch minimalinvasiv erfolgen. Insgesamt wird somit das Infektionsrisiko deutlich gesenkt. Zudem wird die psychische Belastung eines Patienten mit einem elektrische Energie umsetzenden Implantat deutlich reduziert. Ebenso ist die Notwendigkeit von Narkosen somit geringer.

Bevorzugt ist es des Weiteren, das elektrische Energie umsetzende Implantat ausgewählt wird aus der Gruppe, bestehend aus eine Mess-Chip, einem Steuer-Chip, einem Regel-Chip, einer implantierten Wirkstoffdosierungseinheit, einer Mikro-Pumpe und beliebige Kombinationen davon. Damit kann erfindungsgemäß auch ein Online-Monitoring-System oder ein Diagnostik-System betrieben werden. Auch ist es möglich, eine Pumpe für eine kontrollierte Wirkstofffreisetzung zu betreiben. Bevorzugt können auf Grund des hohen Wirkungsgrades auch mehrere Systeme gleichzeitig betrieben werden.

In einem weiteren Aspekt der vorliegenden Erfindung ist eine Vorrichtung zur Durchführung des vorstehend beschriebenen Verfahrens vorgesehen. Im Hinblick auf die körperlichen Merkmale der Vorrichtung wird auf obige Ausführungen zum erfindungsgemäßen Verfahren verwiesen. Insbesondere handelt es sich um eine Vorrichtung, umfassend

(a) eine elektrische Spannungsquelle, welche elektrische Spannung durch die lineare Bewegung eines Kolbens (7) eines Freikolbengenerators (2) erzeugt, wobei der Freikolbengenerator (2) einen Führungszylinder (6), in dem sich ein Kolben (7) mit mindestens einem Permanentmagneten befindet, und mindestens eine Spule (8) umfasst,
der Führungszylinder (6) in ein Blutgefäß (1) eingebracht ist und die mindestens eine Spule (8) sich außerhalb des Blutgefäßes (1) befindet,
der Kolben (7) so ausgebildet ist, dass er innerhalb des Führungszylinders (6) des Freikolbengenerators (2) durch das periodisch pulsierende Blut im Blutgefäß (1) linear relativ in Richtung des jeweiligen

Blutflusses (F) bewegt werden kann, und
in Abhängigkeit dieser linearen Bewegung des Kolbens (7) eine elektrische Spannung in der mindestens einen Spule (8) induziert wird, und
(b) ein elektrische Energie umsetzendes Implantat, umfassend eine Stromsteuerungseinheit, welche so ausgebildet ist, dass sie die elektrische Energie aus der elektrischen Spannungsquelle entnimmt.

In der erfindungsgemäßen Vorrichtung wird der Kolben (7) vorzugsweise formschlüssig im Führungszylinder (6) geführt.

Des Weiteren ist bevorzugt, wenn der Führungszylinder (7) im Wesentlichen axialsymmetrisch im Blutgefäß (1) angeordnet ist und dabei von einem rohrförmigen Gittergerüst (4), welches mindestens eine Halterung (5) zum Halten des Führungszylinders aufweist, gehalten wird.

Das Blutgefäß (1) ist im Allgemeinen eine Vene.

Der anteilige Restdurchmesser des Blutgefäßes (1) erfüllt vorzugsweise die folgende Anforderung:

$$1 > x_{rd} = d/D \geq 0{,}5,$$

worin

- $x_{rd}$ für den anteiligen Restdurchmesser des Blutgefäßes (1), welcher der Strömung des Blutes an der Stelle des Blutgefäßes (1), an der der Führungszylinder (6) des Freikolbengenerators (2) fixiert ist, zur Verfügung steht, steht,
- d für den noch verbleibenden Durchmesser des Blutgefäßes (1), welcher der Strömung des Blutes an der Stelle des Blutgefäßes (1), an der der Führungszylinder (6) des Freikolbengenerators (2) fixiert ist, zur Verfügung steht, steht und
- D für den Gesamtdurchmesser des Blutgefäßes (1) steht, wobei alle Durchmesser Innendurchmesser des Blutgefäßes (1) sind.

Der Kolben (7) des Freikolbengenerators (2) weist vorzugsweise im Wesentlichen eine gerade zylindrische Form mit einer Kreisgrundfläche auf und an der Grundfläche und Deckfläche des Zylinders sind vorzugsweise jeweils eine im Wesentlichen halbkugelförmige Kappe formschlüssig aufgesetzt.

Mindestens eine der im Wesentlichen halbkugelförmigen Kappen weist im Allgemeinen mindestens eine Öffnung auf, wobei die Öffnung im Allgemeinen zur kontrollierten Abgabe mindestens eines Wirkstoffes verwendet wird.

Das elektrische Energie umsetzende Implantat ist vorzugsweise ein Herzschrittmacher. Das elektrische Energie umsetzende Implantat ist vorzugsweise ausgewählt wird aus der Gruppe, bestehend aus eine Mess-Chip, einem Steuer-Chip, einem Regel-Chip, einer implantierten Wirkstoffdosierungseinheit, einer Mikro-Pumpe und beliebige Kombinationen davon.

**Bezugszeichenliste**

- **1** Blutgefäß
- **2** Freikolbengenerator
- **3** Gesamtsystem, welches in das Innere des Blutgefäßes 1 eingebracht ist
- **4** Rohrförmiges Gittergerüst
- **5** Halterung
- **6** Führungszylinder
- **7** Kolben
- **8** Spule
- **9** Feder
- **F** Richtung des Blutflusses

**Beschreibung der Figuren**

Figur 1: zeigt einen Längsschnitt durch ein Blutgefäß 1, in das ein Freikolbengenerator integriert ist. Dabei sind die Halterungen 5 Verstrebungen zwischen der Außenseite des Führungszylinders 6 und dem rohrförmigen Gittergerüst 4. Der Führungszylinder 6 ist im Wesentlichen axialsymmetrisch in das Blutgefäß 1 eingebracht. Der Kolben 7 weist zwei halbkugelförmige Kappen auf.

Figur 2: entspricht im Wesentlichen Figur 1, wobei zusätzlich im Führungszylinder 6 eine mechanische Feder 9 integrieret ist.

**Patentansprüche**

1. Verfahren zur Versorgung eines elektrische Energie umsetzenden Implantates mit elektrischer Energie, umfassend die Schritte:

(a) Bereitstellen einer elektrischen Spannungsquelle mittels Erzeugung elektrischer Spannung durch die lineare Bewegung eines Kolbens (7) eines Freikolbengenerators (2),
wobei der Freikolbengenerator (2) einen Führungszylinder (6), in dem sich ein Kolben (7) mit mindestens einem Permanentmagneten befindet, und mindestens eine Spule (8) umfasst, der Führungszylinder (6) in ein Blutgefäß (1) eingebracht ist,

(b) Entnahme elektrischer Energie von der elektrischen Spannungsquelle aus Schritt (a) durch die Stromsteuerungseinheit des elektrische Energie umsetzenden Implantates,

**dadurch gekennzeichnet, dass**

sich die mindestens eine Spule (8) außerhalb des Blutgefäßes (1) befindet,

der Kolben (7) innerhalb des Führungszylinders (6) des Freikolbengenerators (2) durch das periodisch pulsierende Blut im Blutgefäß (1) linear relativ in Richtung des jeweiligen Blutflusses (F) bewegt wird, und

in Abhängigkeit dieser linearen Bewegung des Kolbens (7) eine elektrische Spannung in der mindestens einen Spule (8) induziert wird.

**2.** Verfahren nach Anspruch 1, wobei der Kolben (7) formschlüssig im Führungszylinder (6) geführt wird.

**3.** Verfahren nach einem der Ansprüche 1 oder 2, wobei der Führungszylinder (7) im Wesentlichen axialsymmetrisch im Blutgefäß (1) angeordnet ist und dabei von einem rohrförmigen Gittergerüst (4), welches mindestens eine Halterung (5) zum Halten des Führungszylinders aufweist, gehalten wird.

**4.** Verfahren nach einem der Ansprüche 1 bis 3, wobei ein anteiliger Restdurchmesser des Blutgefäßes (1) die folgende Anforderung erfüllt:

$$1 > x_{rd} = d/D \geq 0{,}5,$$

worin

$x_{rd}$ für den anteiligen Restdurchmesser des Blutgefäßes (1), welcher der Strömung des Blutes an der Stelle des Blutgefäßes (1), an der der Führungszylinder (6) des Freikolbengenerators (2) fixiert ist, zur Verfügung steht, steht,

d für den noch verbleibenden Durchmesser des Blutgefäßes (1), welcher der Strömung des Blutes an der Stelle des Blutgefäßes (1), an der der Führungszylinder (6) des Freikolbengenerators (2) fixiert ist, zur Verfügung steht, steht und

D für den Gesamtdurchmesser des Blutgefäßes (1) steht, wobei alle Durchmesser Innendurchmesser des Blutgefäßes (1) sind.

**5.** Verfahren nach einem der Ansprüche 1 bis 4, wobei der Kolben (7) des Freikolbengenerators (2) im Wesentlichen eine gerade zylindrische Form mit einer Kreisgrundfläche aufweist und an der Grundfläche und Deckfläche des Zylinders jeweils eine im Wesentlichen halbkugelförmige Kappe formschlüssig aufgesetzt ist.

**6.** Verfahren nach Anspruch 5, worin mindestens eine der im Wesentlichen halbkugelförmigen Kappen mindestens eine Öffnung aufweist.

**7.** Verfahren nach Anspruch 6, worin die mindestens eine Öffnung zur kontrollierten Abgabe mindestens eines Wirkstoffes verwendet wird.

**8.** Verfahren nach einem der Ansprüche 1 bis 7, wobei das elektrische Energie umsetzende Implantat ausgewählt wird aus der Gruppe, bestehend aus eine Mess-Chip, einem Steuer-Chip, einem Regel-Chip, einer implantierten Wirkstoffdosierungseinheit, einer Mikro-Pumpe und beliebige Kombinationen davon.

**9.** Vorrichtung, umfassend

(a) eine elektrische Spannungsquelle, welche elektrische Spannung durch die lineare Bewegung eines Kolbens (7) eines Freikolbengenerators (2) erzeugt,

wobei der Freikolbengenerator (2) einen Führungszylinder (6), in dem sich ein Kolben (7) mit mindestens einem Permanentmagneten befindet, und mindestens eine Spule (8) umfasst, der Führungszylinder (6) in ein Blutgefäß (1) eingebracht ist,

und

(b) ein elektrische Energie umsetzendes Implantat, umfassend eine Stromsteuerungseinheit, welche so ausgebildet ist, dass sie die elektrische Energie aus der elektrischen Spannungsquelle entnimmt,

**dadurch gekennzeichnet, dass**

sich die mindestens eine Spule (8) außerhalb des Blutgefäßes (1) befindet,

der Kolben (7) innerhalb des Führungszylinders (6) des Freikolbengenerators (2) durch das periodisch pulsierende Blut im Blutgefäß (1) linear relativ in Richtung des jeweiligen Blutflusses (F) bewegt wird, und

in Abhängigkeit dieser linearen Bewegung des Kolbens (7) eine elektrische Spannung in der mindestens einen Spule (8) induziert wird.

**10.** Vorrichtung nach Anspruch 9, wobei der Kolben (7) formschlüssig im Führungszylinder (6) geführt wird.

**11.** Vorrichtung nach Anspruch 9 oder 10, wobei ein anteiliger Restdurchmesser des Blutgefäßes (1) die folgende Anforderung erfüllt:

$$1 > x_{rd} = d/D \geq 0,5,$$

worin

$x_{rd}$ für den anteiligen Restdurchmesser des Blutgefäßes (1), welcher der Strömung des Blutes an der Stelle des Blutgefäßes (1), an der der Führungszylinder (6) des Freikolbengenerators (2) fixiert ist, zur Verfügung steht, steht,
d für den noch verbleibenden Durchmesser des Blutgefäßes (1), welcher der Strömung des Blutes an der Stelle des Blutgefäßes (1), an der der Führungszylinder (6) des Freikolbengenerators (2) fixiert ist, zur Verfügung steht, steht und
D für den Gesamtdurchmesser des Blutgefäßes (1) steht, wobei alle Durchmesser Innendurchmesser des Blutgefäßes (1) sind.

**12.** Vorrichtung nach einem der Ansprüche 9 bis 11, wobei der Kolben (7) des Freikolbengenerators (2) im Wesentlichen eine gerade zylindrische Form mit einer Kreisgrundfläche aufweist und an der Grundfläche und Deckfläche des Zylinders jeweils eine im Wesentlichen halbkugelförmige Kappe formschlüssig aufgesetzt ist.

**13.** Vorrichtung nach Anspruch 12, worin mindestens eine der im Wesentlichen halbkugelförmigen Kappen mindestens eine Öffnung aufweist.

**14.** Vorrichtung nach Anspruch 13, worin die mindestens eine Öffnung zur kontrollierten Abgabe mindestens eines Wirkstoffes verwendet wird.

**15.** Vorrichtung nach einem der Ansprüche 9 bis 14, wobei das elektrische Energie umsetzende Implantat ausgewählt wird aus der Gruppe, bestehend aus eine Mess-Chip, einem Steuer-Chip, einem Regel-Chip, einer implantierten Wirkstoffdosierungseinheit, einer Mikro-Pumpe und beliebige Kombinationen davon.

## Claims

**1.** A method for supplying an implant using electric power with electric power, comprising the steps of:

(a) providing an electric voltage source by means of generating electric voltage by the linear movement of a piston (7) of a free piston generator (2),
wherein the free piston generator (2) comprises a guide cylinder (6), in which a piston (7) with at least one permanent magnet is arranged, and at least one coil (8),
the guide cylinder (6) is inserted in a blood vessel (1),
(b) drawing electric energy from the electric voltage source of step (a) by means of the current control unit of the implant using electric energy,
**characterized in that**
the at least one coil (8) is placed outside the blood vessel (1),
the piston (7) within the guide cylinder (6) of the free piston generator (2) is moved in a linearly relative manner into the direction of the respective blood flow (F) by means of the periodically pulsing blood in the blood vessel (1), and in dependence on this linear movement of the piston (7) an electric voltage will be induced in the at least one coil (8).

**2.** A method according to claim 1, wherein the piston (7) is positively guided in the guide cylinder (6).

**3.** A method according to one of the claims 1 or 2, wherein the guide cylinder (6) is essentially arranged axissymmetrically in the blood vessel (1) and is held by means of a tubular lattice frame (4) which comprises at least one bracket (5) for holding the guide cylinder.

**4.** A method according to one of the claims 1 through 3, wherein a proportional residual diameter of the blood vessel (1) meets the following requirement:

$$1 > x_{rd} = d/D \geq 0.5,$$

wherein

$x_{rd}$ represents the proportional residual diameter of the blood vessel (1) which is available for the flow of the blood at the point of the blood vessel (1) at which the guide cylinder (6) of the free piston generator (2) is fixed,
d represents the still remaining diameter of the blood vessel (1) which is available for the flow of the blood at the point of the blood vessel (1) at which the guide cylinder (6) of the free piston generator (2) is fixed, and
D represents the total diameter of the blood vessel (1), wherein all diameters are inner diameters of the blood vessel (1).

**5.** A method according to one of the claims 1 through 4, wherein the piston (7) of the free piston generator (2) essentially comprises a straight cylindrical shape having a circular base surface, and an essentially hemispherical cap is respectively attached in a positive manner on the base surface and the cover surface of the cylinder.

**6.** A method according to claim 5, wherein at least one of the essentially hemispherical caps comprises at least one opening.

**7.** A method according to claim 6, wherein the at least one opening is used for the controlled delivery of at least one active substance.

**8.** A method according to one of the claims 1 through 7, wherein the implant using electric energy is selected from the group consisting of a measuring chip, a controller chip, a regulation chip, an implanted active substance dosing unit, a micro-pump and any combinations thereof.

**9.** A device comprising:

(a) an electric voltage source which generates electric voltage by the linear movement of a piston (7) of a free piston generator (2),
wherein the free piston generator (2) comprises a guide cylinder (6), in which a piston (7) with at least one permanent magnet is arranged, and at least one coil (8),
the guide cylinder (6) is inserted in a blood vessel (1),
and
(b) an implant using electric energy, comprising a current control unit which is configured such that is draws the electric energy from the electric voltage source,
**characterized in that**
the at least one coil (8) is placed outside the blood vessel (1),
the piston (7) within the guide cylinder (6) of the free piston generator (2) is moved in a linearly relative manner into the direction of the respective blood flow (F) by means of the periodically pulsing blood in the blood vessel (1), and
in dependence on this linear movement of the piston (7) an electric voltage will be induced in the at least one coil (8).

**10.** A device according to claim 9, wherein the piston (7) is positively guided in the guide cylinder (6).

**11.** A device according to claim 9 or 10, wherein a proportional residual diameter of the blood vessel (1) meets the following requirement:

$$1 > x_{rd} = d/D \geq 0.5,$$

wherein

$x_{rd}$ represents the proportional residual diameter of the blood vessel (1) which is available for the flow of the blood at the point of the blood vessel (1) at which the guide cylinder (6) of the free piston generator (2) is fixed,
d represents the still remaining diameter of the blood vessel (1) which is available for the flow of the blood at the point of the blood vessel (1) at which the guide cylinder (6) of the free piston generator (2) is fixed, and
D represents the total diameter of the blood vessel (1), wherein all diameters are inner diameters of the blood vessel (1).

**12.** A device according to one of the claims 9 through 11, wherein the piston (7) of the free piston generator (2) essentially comprises a straight cylindrical shape having a circular base surface, and an essentially hemispherical cap is respectively attached in a positive manner on the base surface and the cover surface of the cylinder.

**13.** A device according to claim 13, wherein at least one of the essentially hemispherical caps comprises at least one opening.

**14.** A device according to claim 13, wherein the at least one opening is used for the controlled delivery of at least one active substance.

**15.** A device according to one of the claims 9 through 14, wherein the implant using electric energy is selected from the group consisting of a measuring chip, a controller chip, a regulation chip, an implanted active substance dosing unit, a micro-pump and any combinations thereof.

## Revendications

**1.** Procédé d'alimentation d'un implant utilisant de l'énergie électrique en énergie électrique, comprenant les étapes de :

(a) fournir une source de tension électrique en générant de la tension électrique par moyen du déplacement linéaire d'un piston (7) d'un générateur à piston libre (2),
le générateur à piston libre (2) comprenant un cylindre de guidage (6), dans lequel se trouve un piston (7) comprenant au moins un aimant permanent, et au moins une bobine (8),
le cylindre de guidage (6) étant inséré dans un vaisseau sanguin (1),
(b) prélever de l'énergie électrique à la source de tension électrique de l'étape (a) par moyen de l'unité de commande de courant de l'implant utilisant de l'énergie électrique,
**caractérisé en ce que**
l'au moins une bobine (8) se trouve à l'extérieur

du vaisseau sanguin (1),
le piston (7) à l'intérieur du cylindre de guidage (6) du générateur à piston libre (2) est déplacé de manière relativement linéaire dans la direction du flux sanguin respectif (F) par moyen du sang pulsant périodiquement dans le vaisseau sanguin (1), et
une tension électrique est induite dans l'au moins une bobine (8) en fonction de ce déplacement linéaire du piston (7).

**2.** Procédé selon la revendication 1, dans lequel le piston (7) est guidé par concordance de forme dans le cylindre de guidage (6).

**3.** Procédé selon l'une des revendications 1 ou 2, dans lequel le cylindre de guidage (6) est disposé dans le vaisseau sanguin (1) de manière essentiellement symétrique axialement et il est retenu par moyen d'une ossature grillagée tubulaire (4), qui comprend au moins un support (5) destiné à retenir le cylindre de guidage.

**4.** Procédé selon l'une des revendications 1 à 3, dans lequel un diamètre restant proportionnel du vaisseau sanguin (1) satisfait à la condition suivante :

$$1 > x_{rd} = d/D \geq 0{,}5,$$

dans lequel

$x_{rd}$ représente le diamètre restant proportionnel du vaisseau sanguin (1), qui est disponible pour le flux du sang à l'endroit du vaisseau sanguin (1) où le cylindre de guidage (6) du générateur à piston libre (2) est fixé,
d représente le diamètre du vaisseau sanguin (1), qui reste encore et qui est disponible pour le flux du sang à l'endroit du vaisseau sanguin (1) où le cylindre de guidage (6) du générateur à piston libre (2) est fixé, et
D représente le diamètre total du vaisseau sanguin (1), tous les diamètres étant des diamètres intérieurs du vaisseau sanguin (1).

**5.** Procédé selon l'une des revendications 1 à 4, dans lequel le piston (7) du générateur à piston libre (2) comprend essentiellement une forme cylindrique droite ayant une surface de base circulaire et un capot essentiellement hémisphérique étant respectivement posé par concordance de forme sur la surface de base et la surface supérieure du cylindre.

**6.** Procédé selon la revendication 5, dans lequel au moins un des capots essentiellement hémisphériques comprend au moins une ouverture.

**7.** Procédé selon la revendication 6, dans lequel l'au moins une ouverture est utilisée pour la délivrance contrôlée d'au moins une substance active.

**8.** Procédé selon l'une des revendications 1 à 7, dans lequel l'implant utilisant de l'énergie électrique est sélectionné dans le groupe composé d'une puce de mesure, d'une puce de commande, d'une puce de contrôle, d'une unité de dosage de substance active implantée, d'une micro-pompe et des combinaisons quelconques de celles-ci.

**9.** Dispositif comprenant :

(a) une source de tension électrique, qui génère de la tension électrique par moyen du déplacement linéaire d'un piston (7) d'un générateur à piston libre (2),
le générateur à piston libre (2) comprenant un cylindre de guidage (6), dans lequel se trouve un piston (7) comprenant au moins un aimant permanent, et au moins une bobine (8),
le cylindre de guidage (6) étant inséré dans un vaisseau sanguin (1),
et
(b) un implant utilisant de l'énergie électrique et comprenant une unité de commande de courant, qui est configurée de sorte qu'elle prélève l'énergie électrique à la source de tension électrique,
**caractérisé en ce que**
l'au moins une bobine (8) se trouve à l'extérieur du vaisseau sanguin (1),
le piston (7) à l'intérieur du cylindre de guidage (6) du générateur à piston libre (2) est déplacé de manière relativement linéaire dans la direction du flux sanguin respectif (F) par moyen du sang pulsant périodiquement dans le vaisseau sanguin (1), et
une tension électrique est induite dans l'au moins une bobine (8) en fonction de ce déplacement linéaire du piston (7).

**10.** Dispositif selon la revendication 9, dans lequel le piston (7) est guidé par concordance de forme dans le cylindre de guidage (6).

**11.** Dispositif selon la revendication 9 ou la revendication 10, dans lequel un diamètre restant proportionnel du vaisseau sanguin (1) satisfait à la condition suivante :

$$1 > x_{rd} = d/D \geq 0{,}5,$$

dans lequel

$x_{rd}$ représente le diamètre restant proportionnel du vaisseau sanguin (1), qui est disponible pour le flux du sang à l'endroit du vaisseau sanguin (1) où le cylindre de guidage (6) du générateur à piston libre (2) est fixé,
d représente le diamètre du vaisseau sanguin (1), qui reste encore et qui est disponible pour le flux du sang à l'endroit du vaisseau sanguin (1) où le cylindre de guidage (6) du générateur à piston libre (2) est fixé, et
D représente le diamètre total du vaisseau sanguin (1), tous les diamètres étant des diamètres intérieurs du vaisseau sanguin (1).

**12.** Dispositif selon l'une des revendications 9 à 11, dans lequel le piston (7) du générateur à piston libre (2) comprend essentiellement une forme cylindrique droite ayant une surface de base circulaire et un capot essentiellement hémisphérique étant respectivement posé par concordance de forme sur la surface de base et la surface supérieure du cylindre.

**13.** Dispositif selon la revendication 12, dans lequel au moins un des capots essentiellement hémisphériques comprend au moins une ouverture.

**14.** Dispositif selon la revendication 13, dans lequel l'au moins une ouverture est utilisée pour la délivrance contrôlée d'au moins une substance active.

**15.** Dispositif selon l'une des revendications 9 à 14, dans lequel l'implant utilisant de l'énergie électrique est sélectionné dans le groupe composé d'une puce de mesure, d'une puce de commande, d'une puce de contrôle, d'une unité de dosage de substance active implantée, d'une micro-pompe et des combinaisons quelconques de celles-ci.

Figur 1

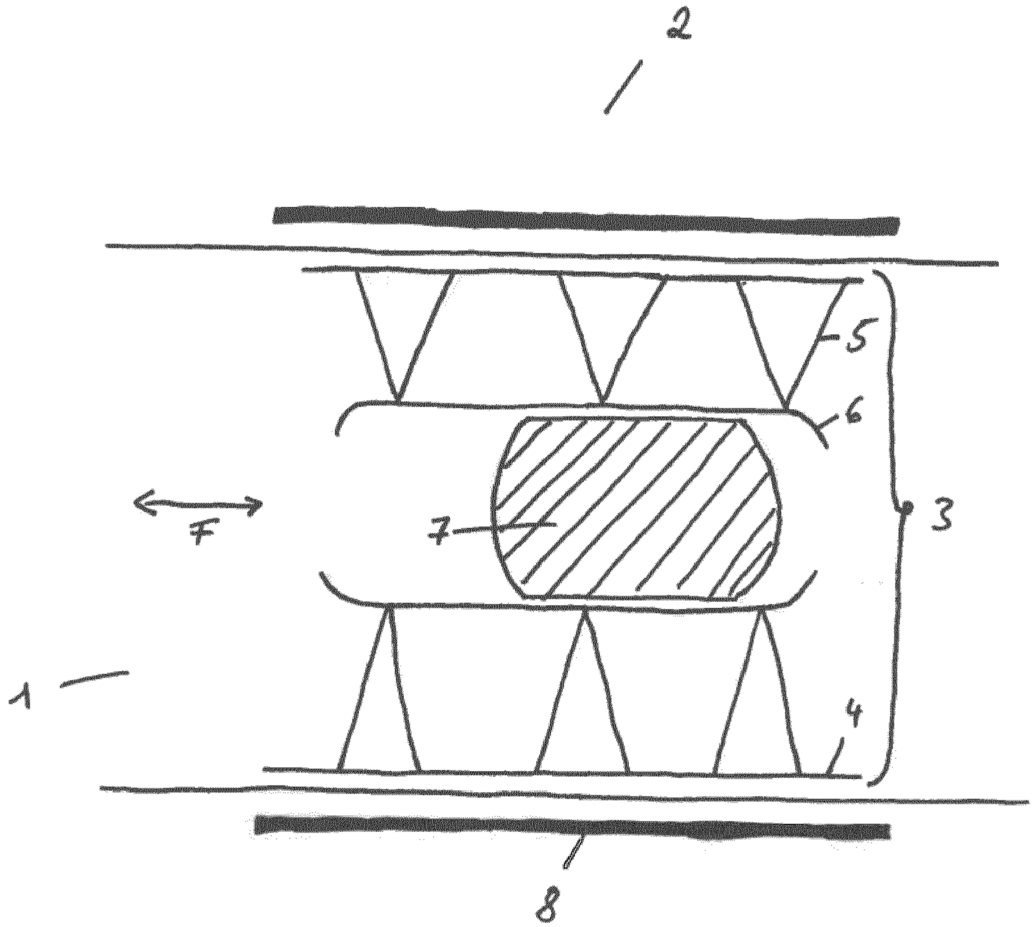

Figur 2

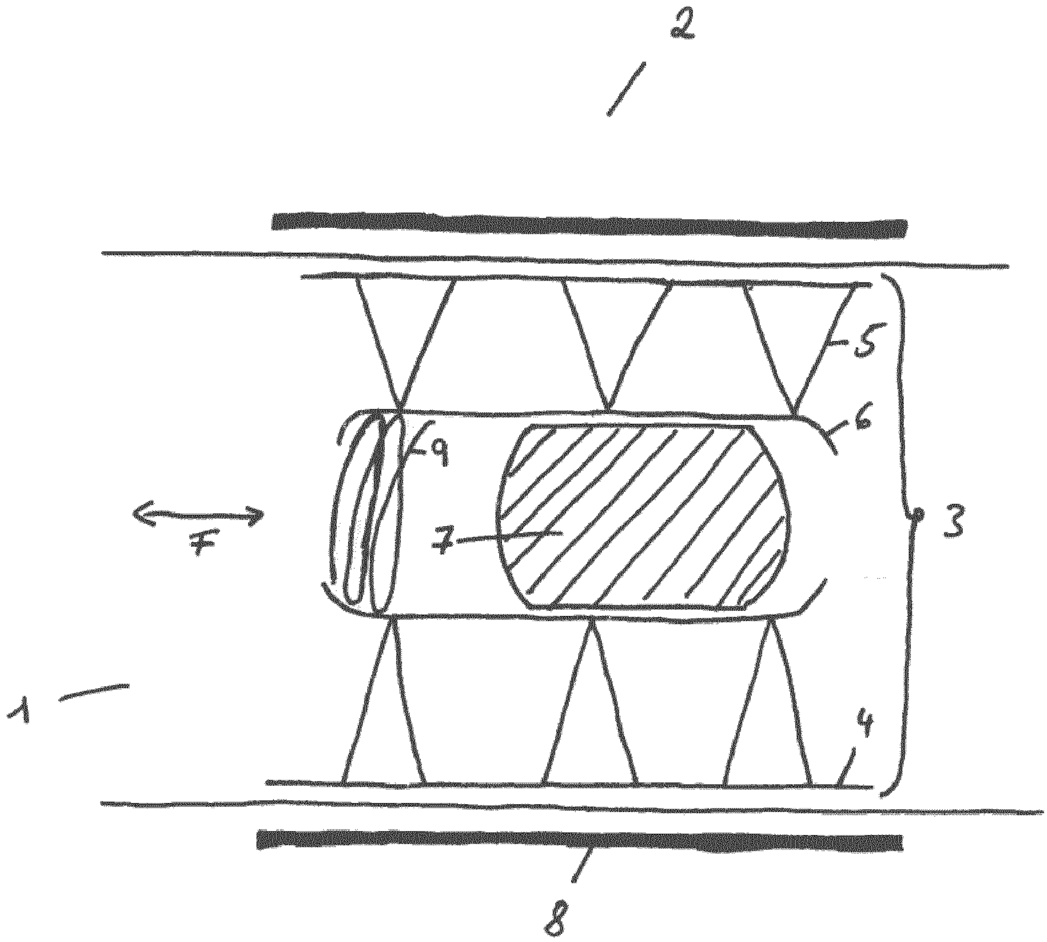

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2008125866 A1
- US 3563245 A
- US 20090171404 A1
- US 4102610 A
- US 3788772 A
- US 5879375 A
- WO 03034893 A2